(19) Europäisches Patentamt / European Patent Office / Office européen des brevets

(11) **EP 2 883 605 A1**

(12) **EUROPEAN PATENT APPLICATION**
published in accordance with Art. 153(4) EPC

(43) Date of publication:
**17.06.2015 Bulletin 2015/25**

(51) Int Cl.:
***B01J 13/12*** *(2006.01)*   ***A61K 9/50*** *(2006.01)*

(21) Application number: **13827439.4**

(22) Date of filing: **08.08.2013**

(86) International application number:
**PCT/JP2013/071509**

(87) International publication number:
**WO 2014/024971 (13.02.2014 Gazette 2014/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.08.2012   JP 2012176100**

(71) Applicant: **Sekisui Chemical Co., Ltd.**
**Osaka-shi, Osaka 530-0047 (JP)**

(72) Inventors:
- **IWAMOTO, Tadashi**
  **Mishima-gun**
  **Osaka 618-0021 (JP)**
- **YAMAUCHI, Hiroshi**
  **Mishima-gun**
  **Osaka 618-0021 (JP)**

(74) Representative: **Hart-Davis, Jason et al**
**Cabinet Beau de Loménie**
**158, rue de l'Université**
**75340 Paris Cedex 07 (FR)**

(54) **MICROCAPSULE-MANUFACTURING PROCESS AND MICROCAPSULES**

(57) The present invention aims to provide a method for producing microcapsules, wherein the method can control the particle size and produce microcapsules excellent in retentivity and releasability of a water-soluble core agent. The present invention also aims to provide a microcapsule excellent in retentivity and releasability of a water-soluble core agent. The present invention provides a method for producing microcapsules comprising the steps of: preparing an emulsion by dispersing an aqueous solution A obtained by dissolving at least an aqueous solvent-soluble polymer and a water-soluble core agent in an aqueous solvent in a non-polar solution B obtained by dissolving an emulsifier or a dispersant in a non-polar medium; and forming a core-shell structure in which the water-soluble core agent is covered with a shell containing the aqueous solvent-soluble polymer by heating the emulsion at a temperature of 20°C to 100°C and/or decompressing the emulsion at a pressure of 0.1 to 0.001 MPa to remove the aqueous solvent, the weight ratio of the aqueous solution A to the non-polar solution B being 1/10 to 1/1.

FIG.1

S3400 10.0kV x3.00k SE    10.0um

EP 2 883 605 A1

**Description**

TECHNICAL FIELD

The present invention relates to a method for producing microcapsules, wherein the method can control the particle size and produce microcapsules excellent in retentivity and releasability of a water-soluble core agent. The present invention also relates to a microcapsule excellent in retentivity and releasability of a water-soluble core agent.

BACKGROUND ART

Microcapsules containing a core agent covered with a shell are used in various fields. For example, in the case of an epoxy resin composition used as an adhesive, sealing agent, coating agent, or the like, a stable one-pack product containing an epoxy resin and a curing agent or a curing accelerator that accelerates curing of the epoxy resin is achieved by use of microcapsules containing a curing agent or a curing accelerator as a core agent covered with a shell to provide a latent effect. Microcapsules are also used in medicinal products. Such microcapsules containing a physiologically active substance or a drug as a core agent covered with a shell. These microcapsules are required to have both the retentivity of a core agent and the releasability (on an as-needed basis) of the core agent.

Although the core agent, such as a physiologically active substance, a drug, a curing agent, or a curing accelerator, is water-soluble in some cases, microcapsules containing a water-soluble core agent or a method for producing the same is unfortunately relatively underdeveloped, compared to the case where the core agent is hydrophobic.

As a microcapsule containing a water-soluble core agent or a method for producing the same, Patent Literature 1 discloses a microcapsule containing a polyvinyl alcohol copolymer, a surfactant, and a drug. As a method for producing microcapsules containing a water-soluble physiologically active substance, Patent Literature 2 discloses a production method including forming a w/o emulsion in which an aqueous solution containing a water-soluble physiologically active substance is the internal water phase and a homogeneous organic solvent solution containing a biodegradable polymer and an oil is the oil phase; and removing the organic solvent.

In the conventional methods disclosed in Patent Literatures 1 and 2, encapsulation is performed by a method such as a spray drying method or a drying-in-liquid method, for example. With the spray drying method, a water-in-oil (w/o) emulsion is sprayed in a dry chamber of a spray dryer and is instantly dried to obtain microcapsules. With the spray drying method, the resulting microcapsules are inhomogeneous in terms of average particle size, shell thickness, and the like, so that the retentivity or releasability of the core agent is unfortunately insufficient. In addition, the particle size is difficult to control.

With the drying-in-liquid method, a water-in-oil (w/o) emulsion is further added to the aqueous phase to form a w/o/w emulsion, and the solvent in the oil phase is dried to obtain microcapsules. The drying-in-liquid method also has a drawback, i.e. , an increase in the average particle size of the resulting microcapsules due to use of a three-phase w/o/w emulsion.

CITATION LIST

- Patent Literature

    Patent Literature 1: WO 2006/106799
    Patent Literature 2: JP-A H11-79976

SUMMARY OF INVENTION

- Technical Problem

The present invention aims to provide a method for producing microcapsules, wherein the method can control the particle size and produce microcapsules excellent in retentivity and releasability of a water-soluble core agent. The present invention also aims to provide a microcapsule excellent in retentivity and releasability of a water-soluble core agent.

- Solution to Problem

The present invention relates to a method for producing microcapsules including the steps of: preparing an emulsion by dispersing an aqueous solution A obtained by dissolving at least an aqueous solvent-soluble polymer and a water-soluble core agent in an aqueous solvent in a non-polar solution B obtained by dissolving an emulsifier or a dispersant in a non-polar medium; and forming a core-shell structure in which the water-soluble core agent is covered with a shell containing the aqueous solvent-soluble polymer by heating the emulsion at a temperature of 20°C to 100°C and/or decompressing the emulsion at a pressure of 0.1 to 0.001 MPa to remove the aqueous solvent, the weight ratio of the aqueous solution A to the non-polar solution B being 1/10 to 1/1.

The present invention is described in detail below.

The present inventors investigated, as a method for forming a core-shell structure in which a water-soluble core agent is covered with a shell, a method for depositing the shell while phase-separating the shell and the water-soluble core agent by heating and/or decompressing a water-in-oil (w/o) emulsion so as to remove a solvent in droplets, in lieu of a conventional method such as the spray drying method or the drying-in-liquid method that uses a w/o/w emulsion.

Specifically, the present inventors found the following: when a method for producing microcapsules includes the step of preparing an emulsion by dispersing an aqueous solution A obtained by dissolving at least an aqueous solvent-soluble polymer and a water-soluble core agent in an aqueous solvent in a non-polar solution B obtained by dissolving an emulsifier or a dispersant in a non-polar medium; and forming a core-shell structure in which the water-soluble core agent is covered with a shell containing the aqueous solvent-soluble polymer by heating and/or decompressing the emulsion under predetermined conditions to remove the aqueous solvent, with the weight ratio of the aqueous solution A to the non-polar solution B in a predetermined range, then the method can produce microcapsules excellent in retentivity and releasability of the core agent even when the core agent is soluble in water. The present inventors also found that the above method for producing microcapsules can control the particle size of microcapsules by adjusting the size of droplets of the aqueous solution A in the emulsion, and the present inventors accomplished the present invention.

The method for producing microcapsules of the present invention first includes the step of preparing an emulsion by dispersing an aqueous solution A obtained by dissolving at least an aqueous solvent-soluble polymer and a water-soluble core agent in an aqueous solvent in a non-polar solution B obtained by dissolving an emulsifier or a dispersant in a non-polar medium.

In this step, the size of droplets of the aqueous solution A in the emulsion is adjusted by adjusting an emulsification method or the like, which in turn enables to control the particle size of microcapsules.

The aqueous solution A is obtained by dissolving at least an aqueous solvent-soluble polymer and a water-soluble core agent in an aqueous solvent.

The aqueous solvent is not particularly limited as long as it can dissolve an aqueous solvent-soluble polymer and a water-soluble core agent at a temperature of about 0°C to 80°C, and is suitably selected in accordance with the aqueous solvent-soluble polymer and the water-soluble core agent. Examples thereof include water, methanol, and a mixed solvent of water and methanol.

The aqueous solvent-soluble polymer is not particularly limited as long as it can be dissolved in an aqueous solvent, and is suitably selected in accordance with the aqueous solvent. The lower limit of the solubility of the aqueous solvent-soluble polymer in an aqueous solvent at 20°C is preferably 0.5% by weight, and the upper limit thereof is preferably 80% by weight, in terms of yield of microcapsules and also in terms of suppressing aggregation of microcapsules caused by the shell softened by the aqueous solvent remaining as residue when the aqueous solvent is removed in the step of forming a core-shell structure. The upper limit of the solubility is more preferably 50% by weight.

Herein, the "solubility of the aqueous solvent-soluble polymer in an aqueous solvent at 20°C" means the maximum amount of the aqueous solvent-soluble polymer at which the solution remains homogeneous when the aqueous solvent-soluble polymer is added to the aqueous solvent at 20°C.

Specific examples of the aqueous solvent-soluble polymer include polyvinyl alcohol, polyvinylphenol, polyvinylpyrrolidone, polyacrylamide, polyacrylic acid, polymethacrylic acid, polyethylene glycol, methylcellulose, hydroxypropyl cellulose, agar, gelatin, poly(acrylic acid-co-acrylamide), and poly(acrylic acid-co-methacrylic acid). These may be used alone or in combination of two or more thereof. Among these, polyvinyl alcohol and methylcellulose are preferred because the properties such as polarity and molecular weight of these polymers can be adjusted. In addition, polyacrylic acid, gelatin, and polyvinylpyrrolidone are also suitably used because the viscosity of these polymers increases only slightly when dissolved in an aqueous solvent and it is thus possible to increase the solids content in the aqueous solution A.

The water-soluble core agent is not particularly limited as long as it can be dissolved in an aqueous solvent. The lower limit of the solubility in an aqueous solvent at 20°C is preferably 0.1% by weight, and the upper limit thereof is preferably 0.5% by weight.

Herein, the "solubility of the water-soluble core agent in an aqueous solvent at 20°C" means the maximum amount of the water-soluble core agent at which the solution remains homogeneous when the water-soluble core agent is added to the aqueous solvent at 20°C.

Examples of the water-soluble core agent include curing agents and/or curing accelerators, foaming agents, adhesives, inks, cosmetic materials, and flavoring agents. For example, in the case where the water-soluble core agent contains a curing agent and/or a curing accelerator, such microcapsules may be suitably used as a latent curing agent and/or a latent curing accelerator. Examples of the curing agent and/or curing accelerator include, but not limited to, hydrazide compounds, amine compounds such as aliphatic polyamine compounds, primary amine compounds, tertiary amine compounds, and imidazole compounds, or phosphorus catalysts. In particular, malonic dihydrazide, dicyandiamide, and 1-benzyl-2-methylimidazole are preferred for their high solubility in an aqueous solvent.

In the case where the water-soluble core agent contains a foaming agent, the resulting microcapsules may be suitably used as microcapsule-type foaming agents that expand by light or heat. Examples of the foaming agent include, but not limited to, tetrazole compounds. Any tetrazole compound may be used, but 3-(1H-tetrazol-5-yl)aniline is preferred.

In the case where the water-soluble core agent contains a cosmetic material, the resulting microcapsules may be suitably used as microcapsule-type cosmetic agents that release the core agent by heat or pressure. Any cosmetic material may be used, but glycerin, hyaluronic acid, and arginine are preferred.

According to the method for producing microcapsules of the present invention, it is possible to produce microcapsules excellent in retentivity and releasability of the core agent, even if the core agent is a water-soluble core agent having high solubility in an aqueous solvent and high polarity (for example, a water-soluble core agent having an SP value of 10 or more).

The "SP value" herein refers to a solubility parameter $\delta$ calculated from formula (A) shown below, using Okitsu's $\Delta F$ and $\Delta v$ values for various atomic groups (Toshinao Okitsu, Setchaku, Kobunshi Kankokai, 1996, Vol. 40, No. 8, pp. 342-350). In the case of mixtures and copolymers, the SP value refers to a solubility parameter $\delta_{mix}$ calculated from formula (B) shown below.

$$\delta \;=\; \Sigma \Delta F / \Sigma \Delta v \quad (A)$$

$$\delta_{mix} \;=\; \phi_1 \delta_1 \;+\; \phi_2 \delta_2 \;+ \cdots \phi_n \delta_n \quad (B)$$

In these formulae, $\Delta F$ represents Okitsu's $\Delta F$ for various atomic groups and $\Delta v$ represents the molar volume $\Delta v$. The symbol $\phi$ represents the volume fraction or molar fraction, with $\phi_1 + \phi_2 + \cdots \phi_n = 1$.

The amount of the water-soluble core agent is not particularly limited. Yet, the lower limit of the amount relative to 100 parts by weight of a raw material constituting the shell is preferably 20 parts by weight, and the upper limit thereof is preferably 150 parts by weight, in terms of weight ratio of the enclosure in the microcapsule as well as in terms of releasability and retentivity of the water-soluble core agent. The lower limit of the amount is more preferably 40 parts by weight, and the upper limit thereof is more preferably 100 parts by weight.

The "raw material constituting the shell" refers to one obtained by adding a crosslinking agent or the like as needed to the aqueous solvent-soluble polymer.

The relationship between the aqueous solvent-soluble polymer and the water-soluble core agent is preferably such that the ratio of the solubility of the water-soluble core agent in an aqueous solvent at 20°C to the solubility of the aqueous solvent-soluble polymer in an aqueous solvent at 20°C (solubility of the water-soluble core agent/solubility of the aqueous solvent-soluble polymer) is more than 1.0. If the solubility ratio is more than 1.0, the aqueous solvent-soluble polymer will be deposited before the water-soluble core agent, so that leakage of the water-soluble core agent into the non-polar solvent b can be suppressed. The solubility ratio is more preferably more than 1.2.

As long as the aqueous solution A is one obtained by dissolving at least an aqueous solvent-soluble polymer and a water-soluble core agent in an aqueous solvent, the aqueous solution A may further contain a crosslinking agent that crosslinks the aqueous solvent-soluble polymer.

In the case of using polyvinyl alcohol as the aqueous solvent-soluble polymer, examples of the crosslinking agent to be added to the aqueous solution A include titanium alkoxide, titanium chelate, and a water-soluble silane coupling agent. In the case of using gelatin as the aqueous solvent-soluble polymer, examples of the crosslinking agent to be added to the aqueous solution A include formaldehyde, glutaraldehyde, titanium chelate, and water-soluble silane coupling agent.

The upper limit of the viscosity of the aqueous solution A is preferably 50 mPa·s, in terms of size of droplets of the aqueous solution A in the emulsion and particle size of microcapsules, as well as in terms of selectively preparing a water-in-oil (w/o) emulsion.

The non-polar solution B is obtained by dissolving an emulsifier or a dispersant in a non-polar medium.

The non-polar medium is not particularly limited, and is suitably selected in accordance with an aqueous solvent.

The relationship between the aqueous solvent and the non-polar medium is preferably such that the non-polar medium has a higher boiling point than the aqueous solvent, and the solubility of the aqueous solvent in the non-polar medium at 20°C is 5% by weight or less. Use of such an aqueous solvent and a non-polar medium enables preparation of a stable emulsion and suppression of coalescence of droplets or the like in the step of forming a core-shell structure, thus enabling to control the particle size of the microcapsules.

The "solubility of the aqueous solvent in a non-polar medium at 20°C" refers to the amount of the aqueous solvent in the non-polar medium when the non-polar medium is analyzed by gas chromatography at 20°C after mixing the non-polar medium and the aqueous solvent and stirring the mixture for one day.

In the case where the aqueous solvent is water (boiling point: 100°C), examples of the non-polar medium include normal paraffinic solvents such as Norpar 13 and Norpar 15 (both available from Exxon Mobil Corporation), naphthenic solvents such as Exxsol D30 and Exxsol D40 (both available from Exxon Mobil Corporation), isoparaffinic solvents such as Isopar G, Isopar H, Isopar L, and Isopar M (all available from Exxon Mobil Corporation), octane, nonane, and decane. These may be used alone or in combination of two or more thereof. Among these, Isopar H and Isopar M are preferred because of their low solubility in water.

The emulsifier is not particularly limited as long as it can be dissolved in a non-polar medium. Yet, the emulsifier preferably has an HLB of 10 or less. An emulsifier having an HLB of 10 or less can stably prepare a water-in-oil (w/o) emulsion, and can suppress formation of an oil-in-water emulsion (o/w) or a multi-layer emulsion (w/o/w).

Specific examples of the emulsifier include sorbitan monolaurate (HLB 8.6), sorbitan monopalmitate (HLB 6.7), sorbitan monostearate (HLB4.7), sorbitan distearate (HLB4.4), sorbitan monooleate (HLB4.3), sorbitan sesquioleate (HLB3.7), sorbitan tristearate (HLB 2.1), and sorbitan trioleate (HLB 1.8).

The lower limit of the amount of the emulsifier added relative to 100 parts by weight of the non-polar medium is preferably 0.05 parts by weight, and the upper limit thereof is preferably 5 parts by weight. If the amount of the emulsifier added is 0.05 parts by weight or more, a water-in-oil (w/o) emulsion can be stably prepared. If the amount of the emulsifier added is 5 parts by weight or less, the size of droplets of the aqueous solution A in the emulsion will be adequate, resulting in microcapsules having an adequate particle size.

The dispersant is not particularly limited as long as it can be dissolved in a non-polar medium. Yet, the dispersant preferably has a molecular weight of 1000 or more. A dispersant having a molecular weight of 1000 or more can further stabilize droplets of the aqueous solution A in the emulsion by steric repulsion.

Specific examples of the dispersant include polydimethylsiloxane, Solsperse 8000, Solsperse 13650, Solsperse 13300, Solsperse 17000, and Solsperse 21000 (all available from the Lubrizol Corporation).

The lower limit of the amount of the dispersant added relative to 100 parts by weight of the non-polar medium is preferably 0.1 parts by weight, and the upper limit thereof is preferably 10 parts by weight. If the amount of the dispersant added is 0.1 parts by weight or more, the dispersant can further stabilize droplets of the aqueous solution A in the emulsion by steric repulsion.

The non-polar solution B may further contain a crosslinking agent that crosslinks the aqueous solvent-soluble polymer.

Examples of the crosslinking agent to be added to the non-polar solution B include, but not limited to, hexamethylene diisocyanate, oil-soluble silane coupling agents, titanium alkoxide, isocyanate-containing polymers, isocyanate-containing oligomers, and silicone alkoxy oligomers.

In preparation of an emulsion by dispersing the aqueous solution A in the non-polar solution B, the non-polar solution B may be added to the aqueous solution A, or the aqueous solution A may be added to the non-polar solution B. Examples of the emulsification method include a method in which a homogenizer is used for stirring, a method in which ultrasonic irradiation is used for emulsification, a method in which an emulsion is formed through microchannels or SPG membranes, a method in which a spray is used for spraying, and a phase-transfer emulsification method.

At this point, the weight ratio of the aqueous solution A to the non-polar solution B is 1/10 to 1/1. If the weight ratio is less than 1/10, the solids content in the emulsion will be low, resulting in a low yield of microcapsules. If the weight ratio is more than 1/1, the volume percent of the aqueous solution A relative to the non-polar solution B will be too high, so that a water-in-oil (w/o) emulsion cannot be selectively prepared, thus unfortunately resulting in a multi-layer emulsion (for example, an o/w/o emulsion). As a result, coalescence of droplets of the aqueous solution A or agglomeration of microcapsules may occur, or the particle size of microcapsules may increase. The lower limit of the weight ratio is preferably 1/4, and the upper limit thereof is preferably 2/3.

The method for producing microcapsules of the present invention subsequently includes the step of forming a core-shell structure in which the water-soluble core agent is covered with a shell containing the aqueous solvent-soluble polymer by heating the emulsion at a temperature of 20°C to 100°C and/or decompressing the emulsion at a pressure of 0.1 to 0.001 MPa to remove the aqueous solvent. Removal of the aqueous solvent enables deposition of the aqueous solvent-soluble polymer while phase-separating the aqueous solvent-soluble polymer and the water-soluble core agent so as to form a core-shell structure.

If the temperature is lower than 20°C or if the pressure is more than 0.1 MPa, removal of the aqueous solvent will take time, thus causing leakage of the water-soluble core agent into the non-polar solution B. If the temperature is higher than 100°C or if the pressure is less than 0.001 MPa, it will cause bumping of the aqueous solvent. Thus, the core-shell structure cannot be formed.

In the step of forming a core-shell structure, it is possible to form pores in the shell of each microcapsule by adjusting temperature and pressure conditions. Thereby, the retentivity and the releasability of the water-soluble core agent can be controlled. The average pore size of such pores tends to increase as the temperature in the step of forming a core-shell structure increases.

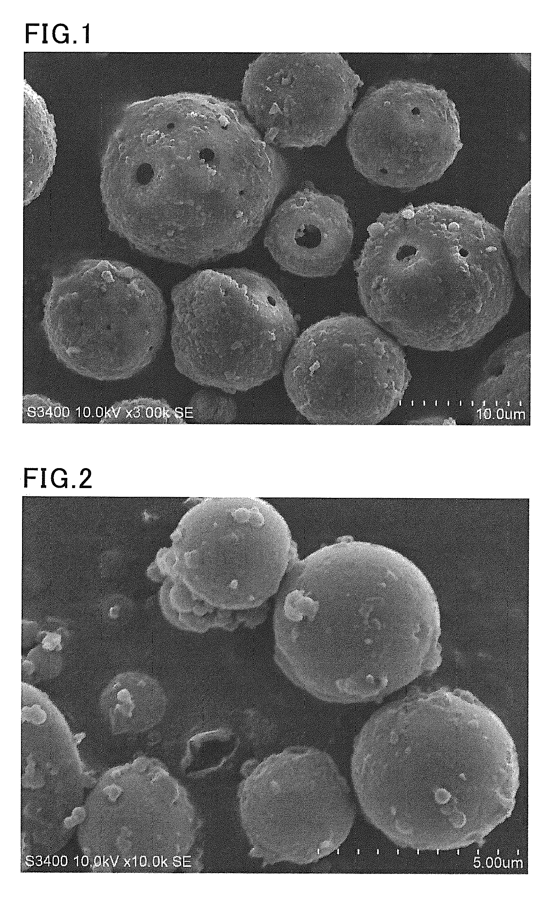

For example, in the case where the aqueous solvent is removed at a temperature of 75°C and a pressure of 0.1 MPa, for example, it results in microcapsules having an average particle size of 5 $\mu$m in which pores having an average pore size of about 2.5 $\mu$m are formed on the surface (see Fig. 1). In contrast, in the case where the aqueous solvent is removed at a temperature of 45°C and a pressure of 0.1 MPa, for example, it results in microcapsules having an average particle size of 5 $\mu$m with no pores on the surface (see Fig. 2).

It should be noted that preferably no pores are present in the shell of the microcapsule in order to improve the retentivity of the water-soluble core agent at room temperature. While the temperature and pressure conditions must be moderated in order to ensure that no pores are present, the evaporation rate of the aqueous solvent is preferably increased in terms of productivity.

Conditions that satisfy the above requirements are as follows: for example, in the case where the aqueous solvent contains water, it is preferred to remove the aqueous solvent at a temperature of 35°C to 70°C and a pressure of 0.1 to 0.01 MPa, and it is more preferred to remove the aqueous solvent under temperature and pressure conditions above the vapor pressure curve of water. In addition, for example, in the case where the aqueous solvent is methanol only, it is preferred to remove the aqueous solvent at temperature of 20°C to 55°C and a pressure of 0.1 to 0.04 MPa, and it is more preferred to remove the aqueous solvent under temperature and pressure conditions above the vapor pressure curve of methanol.

In addition, as for the relationship between the temperature in the step of forming a core-shell structure and the melting point of the aqueous solvent-soluble polymer, the difference between the melting point of the aqueous solvent-soluble polymer and the temperature in step of forming a core-shell structure (i.e., (the melting point of the aqueous solvent-soluble polymer) - (the temperature in the step of forming a core-shell structure)) is preferably higher than 1°C, in terms of suppressing agglomeration of microcapsules. Such agglomeration of microcapsules can also be suppressed by adding a crosslinking agent that crosslinks the aqueous solvent-soluble polymer to the aqueous solution A.

The melting point of the aqueous solvent-soluble polymer can be measured by heating 5 mg of samples from room temperature to 200°C at 5°C/min in nitrogen atmosphere, using a DSC (for example, EXSTAR DSC6200 available from Hitachi High-Tech Science Corporation).

The resulting microcapsules may be further coated, as needed. Examples of the method for further coating the microcapsules include, but not limited to, the drying-in-liquid method in which polystyrene or the like is used, interfacial polycondensation of hexamethylene diisocyanate or the like, polycondensation reaction of a silane coupling agent or titanium alkoxide.

The resulting microcapsules may be repeatedly washed with purified water, and then dried by, for example, vacuum drying.

The method for producing microcapsules of the present invention can produce microcapsules excellent in retentivity and releasability of a water-soluble core agent. In addition, the particle size of the microcapsules can be controlled by adjusting the size of droplets of the aqueous solution A in the emulsion. Further, the retentivity and the releasability of the water-soluble core agent can be controlled by adjusting the temperature and pressure conditions in the step of forming a core-shell structure.

Another aspect of the present invention is a microcapsule obtained by the method for producing microcapsules of the present invention.

As for the shell thickness of the microcapsule of the present invention, the lower limit is preferably 0.05 $\mu$m and the upper limit is preferably 0.8 $\mu$m, in terms of retentivity and releasability of the water-soluble core agent. The lower limit of the shell thickness is more preferably 0.08 $\mu$m, and the upper limit thereof is more preferably 0.5 $\mu$m.

The shell thickness is a value calculated using formulae (1) and (2) shown below. In other words, it is a value determined by subtracting the diameter of the water-soluble core agent calculated from the volume of the microcapsule and the proportion of the enclosure volume from the average particle size of the microcapsules.

```
Shell thickness = {(average particle size of microcapsules) -
(diameter of water-soluble core agent)}/2   (1)
Diameter of water-soluble core agent
```

$$= 2 \times \{(3 \times \text{volume of microcapsule} \times \text{proportion of enclosure volume})/(4 \times \pi)\}^{(1/3)} \quad (2)$$

As for the proportion of the enclosure volume in the microcapsule of the present invention, the lower limit is preferably 15 volume% and the upper limit is preferably 70 volume%, in terms of retentivity and releasability of the water-soluble core agent. The lower limit of the proportion of the enclosure volume is more preferably 25 volume%, and the upper limit thereof is more preferably 50 volume%.

The "proportion of the enclosure volume" herein means a value calculated from formula (3) shown below, using the volume of the microcapsule calculated from the average particle size and the amount of the core agent determined by gas chromatography.

$$\text{Proportion of enclosure volume (\%)} = (\text{amount of water-soluble core agent (\% by weight)}/\text{specific gravity of water-soluble core agent (g/cm}^3))/\text{volume of microcapsule (cm}^3) \quad (3)$$

As for the average particle size of the microcapsules of the present invention, the lower limit is preferably 0.1 $\mu$m and the upper limit is preferably 50.0 $\mu$m, in terms of retentivity of the water-soluble core agent as well as in terms of suppressing leakage of the water-soluble core agent into the non-polar solution B during removal of the aqueous solvent in the step of forming a core-shell structure. The upper limit of the average particle size is more preferably 10.0 $\mu$m.

The "average particle size" herein means the average value of maximum diameters, measured with a caliper, of 50 microcapsules randomly selected from microcapsules observed with a scanning electron microscope at a magnification that enables observation of about 100 microcapsules in one field of view.

The microcapsule of the present invention may or may not have pores on the surface. Although the average pore size of such pores is not particularly limited, the upper limit is preferably 3.0 $\mu$m in terms of retentivity of the water-soluble core agent. The upper limit of the average pore size is more preferably 2.5 $\mu$m.

The "average pore size" means the average value of pore sizes, measured with a caliper, of 25 microcapsules randomly selected from images of 10 fields of view of microcapsules observed with a scanning electron microscope at a magnification that enables observation of about 10 microcapsules in one field of view.

- Advantageous Effects of Invention

The present invention can provide a method for producing microcapsules, wherein the method can control the particle size and produce microcapsules excellent in retentivity and releasability of a water-soluble core agent. The present invention can also provide a microcapsule excellent in retentivity and releasability of a water-soluble core agent.

BRIEF DESCRIPTION OF DRAWINGS

Fig. 1 is an electron micrograph of microcapsules obtained by removing an aqueous solvent at a temperature of 75°C and a pressure of 0.1 MPa according to the method for producing microcapsules of the present invention.
Fig. 2 is an electron micrograph of microcapsules obtained by removing an aqueous solvent at a temperature of 45°C and a pressure of 0.1 MPa according to the method for producing microcapsules of the present invention.

DESCRIPTION OF EMBODIMENTS

The present invention is described in further detail below with reference to examples, but the present invention is not limited to these examples.

(Example 1)

Polyvinyl alcohol (W-24N available from DENKI KAGAKU KOGYO KABUSHIKI KAISHA, solubility in water at 20°C of 16% by weight, melting point of 180°C, amount of 3 parts by weight) as an aqueous solvent-soluble polymer and malonic dihydrazide (SP value of 18.6, solubility in water at 20°C of 45% by weight, amount of 1 part by weight) as a water-soluble core agent were dissolved in water (boiling point of 100°C, solubility in isoparaffinic solvent Isopar H at 20°C of 0.1% by weight or less, amount of 75 parts by weight) to obtain an aqueous solution A (viscosity of the aqueous solution: 29 mPa·s; ratio of the solubility of the water-soluble core agent to the solubility of the aqueous solvent-soluble polymer = 2.8).

Separately, a non-polar solution B containing sorbitan sesquioleate (1% by weight) as an emulsifier in an isoparaffinic solvent (Isopar H available from Exxon Mobil Corporation, boiling point of 179°C) as a non-polar medium was prepared.

Then, the aqueous solution A (79 parts by weight) was added to the non-polar solution B (375 parts by weight), and the mixture was emulsified and dispersed by stirring at 5000 rpm with a homogenizer. Subsequently, the resulting emulsion was heated at 70°C and decompressed under vacuum of 0.1 MPa in a reactor equipped with a decompressor to remove the water, whereby a dispersion of microcapsules having a core-shell structure was obtained. Microcapsules in the microcapsule dispersion obtained were repeatedly washed with cyclohexane, and then vacuum-dried.

(Example 2)

Microcapsules were obtained in the same manner as in Example 1 except that the emulsion was heated at 55°C and decompressed under vacuum of 0.1 MPa in a reactor equipped with a decompressor to remove the water.

(Example 3)

Microcapsules were obtained in the same manner as in Example 1 except that the emulsion was heated at 45°C and decompressed under vacuum of 0.1 MPa in a reactor equipped with a decompressor to remove the water.

(Example 4)

Gelatin (available from Wako Pure Chemical Industries, Ltd. , solubility in water at 20°C of 20% by weight, melting point of 40°C (crosslinking with glutaraldehyde results in disappearance of the melting point), amount of 3 parts by weight) as an aqueous solvent-soluble polymer, a 25% aqueous solution of glutaraldehyde (available from Wako Pure Chemical Industries, Ltd., amount of 1 part by weight), and malonic dihydrazide (SP value of 18.6, solubility in water at 20°C of 45% by weight, amount of 1 part by weight) as a water-soluble core agent were dissolved in water (boiling point of 100°C, solubility in isoparaffinic solvent Isopar H at 20°C of 0.1% by weight or less, amount of 75 parts by weight) to obtain an aqueous solution A (viscosity of the aqueous solution: 13 mPa·s; ratio of the solubility of the water-soluble core agent to the solubility of the aqueous solvent-soluble polymer = 2.3).

Then, microcapsules were obtained in the same manner as in Example 1 except that the above aqueous solution A was used in an amount of 80 parts by weight and the emulsion was heated at 45°C and decompressed under vacuum of 0.1 MPa in a reactor equipped with a decompressor to remove the water.

(Example 5)

The microcapsules (20 parts by weight) obtained in Example 4 were dispersed in Isopar H (200 parts by weight), and hexamethylene diisocyanate (1 part by weight) was added to the dispersion, followed by stirring at 60°C for 24 hours. Microcapsules in the microcapsule dispersion obtained were repeatedly washed with cyclohexane, and then vacuum-dried.

(Example 6)

Gelatin (available from Wako Pure Chemical Industries, Ltd., solubility in water at 20°C of 20% by weight, melting point of 40°C (crosslinking with glutaraldehyde results in disappearance of the melting point), amount of 1.5 parts by weight) as an aqueous solvent-soluble polymer, a 25% aqueous solution of glutaraldehyde (available from Wako Pure Chemical Industries, Ltd., amount of 0.5 parts by weight), and malonic dihydrazide (SP value of 18.6, solubility in water at 20°C of 45% by weight, amount of 0.5 parts by weight) as a water-soluble core agent were dissolved in water (boiling point of 100°C, solubility in isoparaffinic solvent Isopar H at 20°C of 0.1% by weight or less, amount of 35 parts by weight) to obtain an aqueous solution A (viscosity of the aqueous solution: 13 mPa·s; ratio of the solubility of the water-soluble core agent to the solubility of the aqueous solvent-soluble polymer = 2.3).

Microcapsules were obtained in the same manner as in Example 4 except that the above aqueous solution A was used in an amount of 37.5 parts by weight.

(Example 7)

Gelatin (available from Wako Pure Chemical Industries, Ltd., solubility in water at 20°C of 20% by weight, melting point of 40°C (crosslinking with glutaraldehyde results in disappearance of the melting point), amount of 10 parts by weight) as an aqueous solvent-soluble polymer, a 25% aqueous solution of glutaraldehyde (available from Wako Pure Chemical Industries, Ltd., amount of 3.3 parts by weight), and malonic dihydrazide (SP value of 18.6, solubility in water at 20°C of 45% by weight, amount of 3.3 parts by weight) as a water-soluble core agent were dissolved in water (boiling point of 100°C, solubility in isoparaffinic solvent Isopar H at 20°C of 0.1% by weight or less, amount of 233.4 parts by weight) to obtain an aqueous solution A (viscosity of the aqueous solution: 13 mPa·s; ratio of the solubility of the water-soluble core agent to the solubility of the aqueous solvent-soluble polymer = 2.3).
Microcapsules were obtained in the same manner as in Example 4 except that the above aqueous solution A was used in an amount of 250 parts by weight.

(Example 8)

Microcapsules were obtained in the same manner as in Example 4 except that the emulsion was heated at 90°C and decompressed under vacuum of 0.075 MPa in a reactor equipped with a decompressor to remove the water.

(Example 9)

Microcapsules were obtained in the same manner as in Example 4 except that the emulsion was heated at 70°C and decompressed under vacuum of 0.04 MPa in a reactor equipped with a decompressor to remove the water.

(Example 10)

Microcapsules were obtained in the same manner as in Example 4 except that the emulsion was heated at 40°C and decompressed under vacuum of 0.015 MPa in a reactor equipped with a decompressor to remove the water.

(Example 11)

Polyacrylic acid (available from Wako Pure Chemical Industries, Ltd., solubility in water at 20°C of 25% by weight, melting point of 200°C or higher, amount of 3 parts by weight) as an aqueous solvent-soluble polymer and 2-methylimidazole (SP value of 10.8, solubility in water at 20°C of 80% by weight, amount of 1 part by weight) as a water-soluble core agent were dissolved in water (boiling point of 100°C, solubility in isoparaffinic solvent Isopar H at 20°C of 0.1% by weight or less, amount of 75 parts by weight) to obtain an aqueous solution A (viscosity of the aqueous solution: 11 mPa·s; ratio of the solubility of the water-soluble core agent to the solubility of the aqueous solvent-soluble polymer = 3.2).
Then, microcapsules were obtained in the same manner as in Example 1 except that the above aqueous solution A was used and the emulsion was heated at 70°C and decompressed under vacuum of 0.1 MPa in a reactor equipped with a decompressor to remove the water.

(Example 12)

Polyacrylic acid (available from Wako Pure Chemical Industries, Ltd. , solubility in water at 20°C of 25% by weight, melting point of 200°C or higher, amount of 3 parts by weight) as an aqueous solvent-soluble polymer and hexamethylenediamine (SP value of 10.1, solubility in water at 20°C of 30% by weight, amount of 1 part by weight) as a water-soluble core agent were dissolved in water (boiling point of 100°C, solubility in isoparaffinic solvent Isopar H at 20°C of 0.1% by weight or less, amount of 75 parts by weight) to obtain an aqueous solution A (viscosity of the aqueous solution: 10 mPa·s; ratio of the solubility of the water-soluble core agent to the solubility of the aqueous solvent-soluble polymer = 1.2).
Microcapsules were obtained in the same manner as in Example 1 except that the above aqueous solution A was used and the emulsion was heated at 70°C and decompressed under vacuum of 0.1 MPa in a reactor equipped with a decompressor to remove the water.

(Example 13)

Polyvinylpyrrolidone (K-30 available fromDai-ichi Kogyo Seiyaku Co.,Ltd., solubility in methanol at 20°C of 40% by weight, melting point of 160°C, amount of 3 parts by weight) as an aqueous solvent-soluble polymer and 3-(1H-tetrazol-5-yl)aniline (SP value of 14.9, solubility in methanol at 20°C of 60% by weight, amount of 1 part by weight) as a water-soluble core agent were dissolved in methanol (boiling point of 65°C, solubility in isoparaffinic solvent Isopar H at 20°C of 0.6% by weight, amount of 75 parts by weight) to obtain an aqueous solution A (viscosity of the aqueous solution: 9 mPa·s; ratio of the solubility of the water-soluble core agent to the solubility of the aqueous solvent-soluble polymer = 1.5).

Then, microcapsules were obtained in the same manner as in Example 1 except that the above aqueous solution A was used and the emulsion was heated at 30°C and decompressed under vacuum of 0.1 MPa in a reactor equipped with a decompressor to remove the methanol.

(Example 14)

Polyvinylpyrrolidone (K-30 available from Dai-ichi Kogyo Seiyaku Co.,Ltd., solubility in methanol at 20°C of 40% by weight, melting point of 160°C, amount of 3 parts by weight) as an aqueous solvent-soluble polymer and 1-benzyl-2-methylimidazole (SP value of 10.6, solubility in methanol at 20°C of 50% by weight, amount of 1 part by weight) as a water-soluble core agent were dissolved in methanol (boiling point of 65°C, solubility in isoparaffinic solvent Isopar H at 20°C of 0.6% by weight, amount of 75 parts by weight) to obtain an aqueous solution A (viscosity of the aqueous solution: 9 mPa·s; ratio of the solubility of the water-soluble core agent to the solubility of the aqueous solvent-soluble polymer = 1.3).

Then, microcapsules were obtained in the same manner as in Example 1 except that the above aqueous solution A was used and the emulsion was heated at 30°C and decompressed under vacuum of 0.1 MPa in a reactor equipped with a decompressor to remove the methanol.

(Example 15)

Gelatin (available from Wako Pure Chemical Industries, Ltd., solubility in water/methanol (50/50) at 20°C of 8% by weight, melting point of 40°C (crosslinking with glutaraldehyde results in disappearance of the melting point), amount of 3 parts by weight) as an aqueous solvent-soluble polymer, a 25% aqueous solution of glutaraldehyde (available from Wako Pure Chemical Industries, Ltd., amount of 1 part by weight), and 3-(1H-tetrazol-5-yl)aniline (SP value of 14.9, solubility in water/methanol (50/50) at 20°C of 20% by weight, amount of 1 part by weight) as a water-soluble core agent were dissolved in a mixed solvent of water (boiling point of 100°C, solubility in isoparaffinic solvent Isopar H at 20°C of 0.1% by weight or less, amount of 35.25 parts by weight) and methanol (boiling point of 65°C, solubility in isoparaffinic solvent Isopar H at 20°C of 0.6% by weight, amount of 35.25 parts by weight) to obtain an aqueous solution A (viscosity of the aqueous solution: 15 mPa·s; ratio of the solubility of the water-soluble core agent to the solubility of the aqueous solvent-soluble polymer = 2.5).

Then, microcapsules were obtained in the same manner as in Example 1 except that the above aqueous solution A was used in an amount of 80 parts by weight and the emulsion was heated at 70°C and decompressed under vacuum of 0.1 MPa in a reactor equipped with a decompressor to remove the methanol.

(Comparative Example 1)

Preparation was performed in the same manner as in Example 1 except that the emulsion was heated at 120°C and decompressed under vacuum of 0.1 MPa in a reactor equipped with a decompressor to remove the water. However, no core-shell structures were formed (no capsules were formed).

(Comparative Example 2)

Gelatin (available from Wako Pure Chemical Industries, Ltd., solubility in water at 20°C of 20% by weight, amount of 16 parts by weight) as an aqueous solvent-soluble polymer, a 25% aqueous solution of glutaraldehyde (available from Wako Pure Chemical Industries, Ltd. , amount of 5.3 parts by weight), and malonic dihydrazide (SP value of 18.6, solubility in water at 20°C of 45% by weight, amount of 5.3 parts by weight) as a water-soluble core agent were dissolved in water (boiling point of 100°C, solubility in isoparaffinic solvent Isopar H at 20°C of 0.1% by weight or less, amount of 373.4 parts by weight) to obtain an aqueous solution A (viscosity of the aqueous solution: 11 mPa·s; ratio of the solubility of the water-soluble core agent to the solubility of the aqueous solvent-soluble polymer = 2.3).

The aqueous solution A (400 parts by weight) was added to the non-polar solution B (375 parts by weight), and the mixture was emulsified and dispersed by stirring at 5000 rpm with a homogenizer. As a result, a multi-layer emulsion was formed, resulting in a viscous emulsion. The water was removed in the same manner as in Example 4, but no core-shell structured microcapsules were obtained (no capsules were formed).

(Comparative Example 3)

Gelatin (available from Wako Pure Chemical Industries, Ltd., solubility in water at 20°C of 20% by weight, amount of 1.2 parts by weight) as an aqueous solvent-soluble polymer, a 25% aqueous solution of glutaraldehyde (available from Wako Pure Chemical Industries, Ltd. , amount of 0.4 parts by weight), and malonic dihydrazide (SP value of 18.6, solubility in water at 20°C of 45% by weight, amount of 0.4 parts by weight) as a water-soluble core agent were dissolved in water (boiling point of 100°, solubility in isoparaffinic solvent Isopar H at 20°C of 0.1% by weight or less, amount of 28 parts by weight) to obtain an aqueous solution A (viscosity of the aqueous solution: 11 mPa·s; ratio of the solubility of the water-soluble core agent to the solubility of the aqueous solvent-soluble polymer = 2.3).

The aqueous solution A (30 parts by weight) was added to the non-polar solution B (375 parts by weight), and the mixture was emulsified and dispersed by stirring at 5000 rpm with a homogenizer. Subsequently, the water was removed in the same manner as in Example 4, and core-shell structured microcapsules were obtained. However, the yield of the resulting microcapsules was very low.

(Comparative Example 4)

Polyvinyl alcohol (KH-20 available from the Nippon Synthetic Chemical Industry Co., Ltd., solubility in water at 20°C of 16% by weight, amount of 3 parts by weight) as an aqueous solvent-soluble polymer and malonic dihydrazide (SP value of 18.6, solubility in water at 20°C of 45% by weight, amount of 1 part by weight) as a water-soluble core agent were dissolved in water (boiling point of 100°C, amount of 75 parts by weight) to obtain an aqueous solution. This solution was spray-dried at 115°C and 0.1 MPa with a spray dryer, whereby core-shell structured microcapsules were obtained.

(Comparative Example 5)

Sodium alginate (available from Wako Pure Chemical Industries, Ltd., amount of 3 parts by weight) and malonic dihydrazide (SP value of 18.6, solubility in water at 20°C of 45% by weight, amount of 1 part by weight) as a water-soluble core agent were dissolved in water (75 parts by weight) to obtain an aqueous solution. This aqueous solution was added dropwise to water (150 parts by weight) containing calcium chloride (3 parts by weight) dissolved therein to obtain a dispersion of microcapsules (the shell contains calcium alginate). Microcapsules in the microcapsule dispersion obtained were repeatedly washed with cyclohexane, and then vacuum-dried.

<Evaluation>

The microcapsules obtained in the examples and the comparative examples were evaluated as follows. Table 1 shows the results.

(1) Measurement of average particle size

Microcapsules were observed with a scanning electron microscope at a magnification that enables observation of about 100 microcapsules in one field of view. Then, the maximum diameters of 50 randomly selected microcapsules were measured with a caliper, and the average value was determined as the average particle size.

(2) Measurement of average pore size

Microcapsules were observed with a scanning electron microscope at a magnification that enables observation of about 10 microcapsules in one field of view, and images of 10 fields of view were provided. Then, the pore sizes of 25 randomly selected microcapsules were measured with a caliper, and the average value was determined as the average pore size. The pores were observed on the surface of the microcapsules obtained only in Examples 1 and 2.

(3) Shell thickness

Using the proportion of the enclosure volume calculated from formula (3) shown below, the diameter of the water-soluble core agent was calculated from formula (2) shownbelow. Further, using the diameter of the water-soluble core agent calculated, the shell thickness was calculated from formula (1) shown below.

```
Shell thickness = {(average particle size of microcapsules) -
(diameter of water-soluble core agent)}/2    (1)
```

Diameter of water-soluble core agent
= 2 × { (3 × volume of microcapsule × proportion of enclosure volume)/(4 × π) }$^{(1/3)}$    (2)

Proportion of enclosure volume (%) = (amount of water-soluble core agent (% by weight)/specific gravity of water-soluble core agent (g/cm$^3$))/volume of microcapsule (cm$^3$)    (3)

The volume of the microcapsule was calculated using the average particle size, and the amount of the water-soluble core agent was determined by gas chromatography.

(4) Retention ratio of water-soluble core agent

The microcapsules (1.0 g) were dispersed in methyl ethyl ketone (100 mL) and the obtained dispersion was stirred at room temperature for 10 days. Subsequently, the microcapsules were removed by filtering. The methyl ethyl ketone in the obtained filtrate was removed by vacuum evaporation. Thereby the amount of the water-soluble core agent dissolved into the methyl ethyl ketone was measured, and the retention ratio of the water-soluble core agent was calculated from formula (4) shown below.

Retention ratio of water-soluble core agent = 100 - ((amount of water-soluble core agent dissolved into methyl ethyl ketone)/(amount of water-soluble core agent encapsulated in capsules) × 100) (% by weight)    (4)

(5) Release ratio of water-soluble core agent

The microcapsules (1.0 g) were dispersed in ethanol (100 mL) and the obtained dispersion was stirred at 40°C for 10 days. Subsequently, the microcapsules were removed by filtering. The ethanol in the obtained filtrate was removed by vacuum evaporation. Thereby the amount of dissolved matter in the ethanol was measured, and the release ratio of the water-soluble core agent was calculated from formula (5) shown below.

Release ratio of water-soluble core agent = (amount of dissolved matter in ethanol)/(amount of water-soluble core agent encapsulated in microcapsules) × 100 (% by weight)    (5)

[Table 1]

| | Step of preparing an emulsion | | | Step of forming a core-shell structure | | Additional coating layer | Average particle size (µm) | Average pore size (µm) | Shell thickness (µm) | Retention ratio (wt%) | Release ratio (wt%) |
| | Aqueous solution A | | Aqueous solution A/non-polar solution B | Temp. (°C) | Pressure (MPa) | | | | | | |
| | Raw material constituting the shell | Water-soluble core agent | | | | | | | | | |
| Example 1 | Polyvinyl alcohol | Malonic dihydrazide | 79/375 | 70 | 0.1 | - | 1.6 | 2.5 | 0.30 | 88 | 64 |
| Example 2 | | | | 55 | 0.1 | - | 2.1 | 0.1 | 0.38 | 95 | 45 |
| Example 3 | | | | 45 | 0.1 | - | 1.9 | - | 0.35 | 98 | 32 |
| Example 4 | Gelatin/glutaraldehyde | Malonic dihydrazide | 80/375 | 45 | 0.1 | - | 1.2 | - | 0.23 | 90 | 78 |
| Example 5 | | | 80/375 | | | Hexamethylene diisocyanate | 1.6 | - | - | 99 | 14 |
| Example 6 | | | 37.5/375 | | | - | 1.1 | - | 0.21 | 80 | 49 |
| Example 7 | | | 250/375 | | | - | 2.2 | - | 0.42 | 84 | 35 |
| Example 8 | Gelatin/glutaraldehyde | Malonic dihydrazide | 80/375 | 90 | 0.075 | - | 3.1 | - | 0.49 | 83 | 33 |
| Example 9 | | | | 70 | 0.04 | - | 2.6 | - | 0.39 | 80 | 32 |
| Example 10 | | | | 40 | 0.015 | - | 2.5 | - | 0.37 | 84 | 32 |
| Example 11 | Polyacrylic acid | 2-Methylimidazole | 79/375 | 70 | 0.1 | - | 1.4 | - | 0.22 | 96 | 186¡※ |
| Example 12 | | Hexamethylenediamine | | | | - | 2.3 | - | 0.39 | 94 | 137¡※ |
| Example 13 | Polyvinylpyrrolidone | 3-(1H-tetrazol-5-yl)aniline | 79/375 | 30 | 0.1 | - | 4.6 | - | 0.50 | 89 | 283¡※ |
| Example 14 | | 1-Benzyl-2-methylimidazole | | | | - | 8.2 | - | 0.79 | 79 | 317¡※ |
| Example 15 | Gelatin/glutaraldehyde | 3-(1H-tetrazol-5-yl)aniline | 80/375 | 70 | 0.1 | - | 3.0 | - | 0.44 | 81 | 38 |

EP 2 883 605 A1

(continued)

| | Step of preparing an emulsion | | | Step of forming a core-shell structure | | Additional coating layer | Average particle size ($\mu$m) | Average pore size ($\mu$m) | Shell thick-ness ($\mu$m) | Retention ratio (wt%) | Release ratio (wt%) |
|---|---|---|---|---|---|---|---|---|---|---|---|
| | Aqueous solution A | | Aqueous solution A/ non-polar solution B | Temp. (°C) | Pressure (MPa) | | | | | | |
| | Raw material constituting the shell | Water-soluble core agent | | | | | | | | | |
| Comparative Example 1 | Polyvinyl alcohol | Malonic dihydrazide | 79/375 | 120 | 0.1 | - | No capsules were formed | | | | |
| Comparative Example 2 | Gelatin/glutaraldehyde | Malonic dihydrazide | 400/375 | 45 | 0.1 | - | No capsules were formed | | | | |
| Comparative Example 3 | | | 30/375 | | | - | 0.9 | - | 0.19 | 69 | 45 |
| Comparative Example 4 | Polyvinyl alcohol | Malonic dihydrazide | - | - | - | - | 6.8 | - | - | 98 | 1 |
| Comparative Example 5 | Calcium alginate | Malonic dihydrazide | - | - | - | - | 1000 or more | - | - | 99 | 1 |

※ The shell also dissolves in ethanol.

EP 2 883 605 A1

INDUSTRIAL APPLICABILITY

The present invention can provide a method for producing microcapsules, wherein the method can control the particle size and produce microcapsules excellent in retentivity and releasability of a water-soluble core agent. The present invention can also provide a microcapsule excellent in retentivity and releasability of a water-soluble core agent.

**Claims**

1. A method for producing microcapsules comprising the steps of:

   preparing an emulsion by dispersing an aqueous solution A obtained by dissolving at least an aqueous solvent-soluble polymer and a water-soluble core agent in an aqueous solvent in a non-polar solution B obtained by dissolving an emulsifier or a dispersant in a non-polar medium; and
   forming a core-shell structure in which the water-soluble core agent is covered with a shell containing the aqueous solvent-soluble polymer by heating the emulsion at a temperature of 20°C to 100°C and/or decompressing the emulsion at a pressure of 0.1 to 0.001 MPa to remove the aqueous solvent,
   the weight ratio of the aqueous solution A to the non-polar solution B being 1/10 to 1/1.

2. A microcapsule obtained by the method for producing microcapsules defined in claim 1.

EP 2 883 605 A1

## FIG.1

S3400 10.0kV x3.00k SE          10.0um

## FIG.2

S3400 10.0kV x10.0k SE          5.00um

## INTERNATIONAL SEARCH REPORT

| International application No. |
|---|
| PCT/JP2013/071509 |

**A. CLASSIFICATION OF SUBJECT MATTER**
*B01J13/12*(2006.01)i, *A61K9/50*(2006.01)i

According to International Patent Classification (IPC) or to both national classification and IPC

**B. FIELDS SEARCHED**

Minimum documentation searched (classification system followed by classification symbols)
B01J13/12, A61K9/50

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2000-26283 A (The Nisshin Oil Mills, Ltd.), 25 January 2000 (25.01.2000), claim 1; paragraphs [0007], [0013] to [0015], [0022] to [0023], [0032] to [0043] (Family: none) | 1,2 |
| A | JP 2002-301357 A (Yoshinobu KAWANO), 15 October 2002 (15.10.2002), paragraphs [0020] to [0023] (Family: none) | 1,2 |

☐ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 18 October, 2013 (18.10.13) | 29 October, 2013 (29.10.13) |

| Name and mailing address of the ISA/<br>Japanese Patent Office | Authorized officer |
|---|---|
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006106799 A

- JP H1179976 A

**Non-patent literature cited in the description**

- **TOSHINAO OKITSU, SETCHAKU.** *Kobunshi Kankokai,* 1996, vol. 40 (8), 342-350